# EUROPÄISCHE PATENTSCHRIFT

(11) **EP 0 884 304 B1**
(45) Veröffentlichungstag und Bekanntmachung des Hinweises auf die Patenterteilung: **04.07.2001**
(21) Anmeldenummer: 98115670.6
(22) Anmeldetag: 12.10.1995
(51) Int. Cl.: C07C 231/02

(54) **Verfahren zur Herstellung von N-(4-Fluor-phenyl)-N-isopropyl-chloracetamid**
Process for the preparation of F-(4-Fluoro-phenyl)-N-isopropyl-chloracetamide
Procédé pour la préparation du N-(Fluoro-4-phényl)-N-isopropyl-chloracétamide

(30) Priorität: 25.10.1994 DE 4438001
(43) Veröffentlichungstag der Anmeldung: 16.12.1998
(62) Teilanmeldung aus: 95116098.5
(73) Patentinhaber: BAYER AG, 51368 Leverkusen (DE)
(72) Erfinder: Lantzsch, Reinhard, Dr., 42115 Wuppertal (DE); Förster, Heinz, DR, 56337 Kadenbach (DE); Schmidt, Thomas, Dr., 42781 Haan (DE); Steinbeck, Karl, Dr, 51399 Burscheid (DE); Dollinger, Markus, Dr., Kansas 66213 (US); Santel, Hans-Joachim, Dr., 51371 Leverkusen (DE)

(56) Entgegenhaltungen:
- FR-A- 2 318 861
- US-A- 4 418 021
- EUGEN MÜLLER: "Methoden der organischen chemie, Band XI/2" , GEORG THIEME VERLAG , STUTTGART XP002086537 * Seite 13, letzter Absatz *
- J. SPEZIALE ET AL.: "Preparation of some new 2-chloroacetamides" JOURNAL OF THE AMERICAN CHEMICAL SOCIETY., Bd. 78, Nr. 11, 8. Juni 1956, Seiten 2556-2559, XP002086481 DC US

## Beschreibung

Die Erfindung betrifft ein neues Verfahren zur Herstellung der bekannten Verbindung N-(4-Fluor-phenyl)-N-isopropyl-chloracetamid, die als Wirkstoff in selektiv-herbiziden Mitteln verwendet werden kann.

N-(4-Fluor-phenyl)-N-isopropyl-chloracetamid ist bereits als Zwischenprodukt für Agrochemikalien bekannt geworden (vgl. DE-A-2633159, US-A-4140774, US-A-4418021); die Verwendung der genannten Verbindung als Herbizid ist jedoch bisher nicht bekannt geworden.

Als Verfahren zur Herstellung der Verbindung der Formel (I) ist bisher die Umsetzung von N-Isopropyl-4-fluor-anilin mit Chloracetylchlorid in Gegenwart von Pyridin bekannt (vgl. US-P 4453965). Die Rückgewinnung von Pyridin aus dem bei dieser Umsetzung gebildeten Pyridin-Hydrochlorid ist jedoch technisch nur in sehr aufwendiger Weise durchzuführen.

Weiter ist bekannt, daß die ähnliche Verbindung N-(Isopropyl-phenyl)-chloracetamid durch Umsetzung von N-Isopropyl-anilin mit Chloressigsäure und Phosphorylchlorid oder Phosphortrichlorid hergestellt werden kann (vgl. US-P 3960948). Bei diesem Verfahren sind jedoch die Ausbeuten nicht ganz befriedigend und man muß phosphorhaltige Abwässer mittels einer Phosphatfällung entsorgen.

Die Chloracetylierung von Amilinderivaten mit Chloracetylchlorid ohne Zusatz eines Säurebindemittels ist bereits bekannt (vgl. FR-A-2318861; J.A.C.S, Bd 78, 1956, Seiten 2556-59 ; Houben-Weyl, Bd XI/2, Seite 13).

Es wurde nun gefunden, daß man die Verbindung der Formel (I) 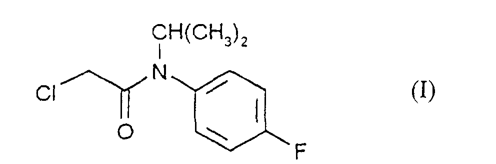 in sehr guten Ausbeuten erhält, wenn man N-Isopropyl-4-fluor-anilin mit Chloracetylchlorid in Gegenwart eines inerten Verdünnungsmittels, wie z.B. Toluol, ohne Zusatz eines Säurebindemittels, in etwa äquimolaren Mengen bei Temperaturen zwischen 20°C und 150°C umsetzt.

Bei dieser Verfahrensweise entsteht kein Abwasser und der freigesetzte Chlorwasserstoff kann wieder für andere Zwecke verwendet werden.

Der Reaktionsablauf bei der Synthese der Verbindung der Formel (I) kann durch das folgende Formelschema skizziert werden:

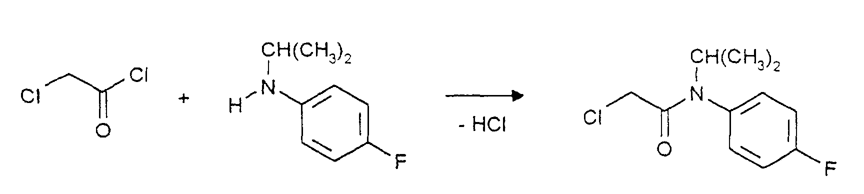

Die zur Herstellung der Verbindung der Formel (I) benötigten Ausgangsstoffe sind bekannte Chemikalien.

Die Verbindung der Formel (I) kann in Defoliants, Desiccants, Krautabtötungsmitteln und insbesondere in Unkrautvernichtungsmitteln verwendet werden. Unter Unkraut im weitesten Sinne sind alle Pflanzen zu verstehen, die an Orten aufwachsen, wo sie unerwünscht sind.

### Herstellungsbeispiel:

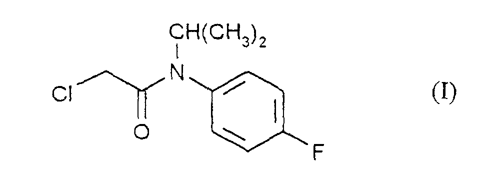

15,3 g (0,1 Mol) 4-Fluor-N-isopropyl-anilin werden in 100 ml Toluol gelöst und zu dieser Lösung werden bei 60°C unter Rühren 11,3 g (0,1 Mol) Chloracetylchlorid tropfenweise gegeben. Die Mischung wird dann 90 Minuten zum Sieden erhitzt; dann wird nach leichtem Abkühlen das Lösungsmittel im Wasserstrahlvakuum und schließlich im Ölpumpenvakuum sorgfältig abdestilliert.

Man erhält 22,9 g (96,5% der Theorie) N-(4-Fluor-phenyl)-N-isopropyl-chloracetamid als klaren flüssigen Rückstand vom Brechungsindex 1,5210.

## Patentansprüche

1. Verfahren zur Herstellung von N-(4-Fluor-phenyl)-N-isopropyl-chloracetamid der Formel (I) 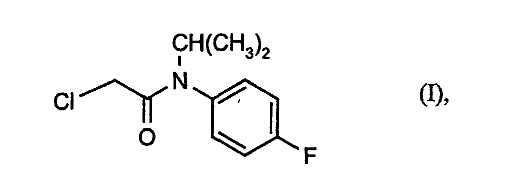 dadurch gekennzeichnet, daß man N-Isopropyl-4-fluor-anilin mit Chloracetylchlorid in Gegenwart eines inerten Verdünnungsmittels, ohne Zusatz eines Säurebindemittels in etwa äquimolaren Mengen bei Temperaturen zwischen 20°C und 150°C umsetzt.

2. Verfahren nach Anspruch 1, dadurch gekennzeichnet, daß man als inertes Verdünnungsmittel Toluol einsetzt.

3. Verfahren nach Anspruch 2, dadurch gekennzeichnet, daß man die Umsetzung bei Temperaturen zwischen 60°C und der Siedetemperatur von Toluol (ca. 111°C bei Normaldruck) durchführt.

## Revendications

1. Procédé pour la préparation du N-(4-fluorophényl)-N-isopropyl-chloracétamide répondant à la formule (I) 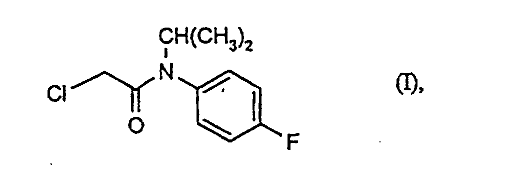 caractérisé en ce qu'on fait réagir de la N-isopropyl-4-fluoro-aniline avec du chlorure de chloracétyle en présence d'un diluant inerte, sans ajouter un agent neutralisant ou fixant les acides dans des quantités approximativement équimolaires à des températures entre 20°C et 150°C.

2. Procédé selon la revendication 1, caractérisé en ce qu'on met en oeuvre, à titre de diluant inerte, du toluène.

3. Procédé selon la revendication 2, caractérisé en ce qu'on effectue la mise en réaction à des températures entre 60°C et la température d'ébullition du toluène (environ 111°C sous pression normale).

## Claims

1. Process for the preparation of N-(4-fluorophenyl)-N-isopropyl-chloroacetamide, of the formula (I) 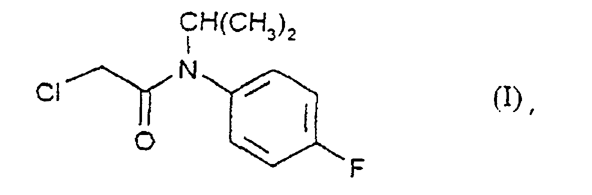 characterized in that N-isopropyl-4-fluoro-anilin is reacted with chloroacetyl chloride in approximately equimolar amounts at temperatures between 20°C and 150°C in the presence of an inert diluent without addition of an acid binder.

2. Process according to Claim 1, characterized in that toluene is employed as inert diluent.

3. Process according to Claim 2, characterized in that the reaction is carried out at temperatures between 60°C and the boiling point of toluene (approx. 111°C under atmospheric pressure).
